(19) Europäisches Patentamt / European Patent Office / Office européen des brevets

(11) **EP 2 977 466 B1**

(12) **EUROPEAN PATENT SPECIFICATION**

(45) Date of publication and mention
of the grant of the patent:
**19.09.2018 Bulletin 2018/38**

(51) Int Cl.:
***C12Q 1/68*** *(2018.01)*      ***G06F 19/18*** *(2011.01)*

(21) Application number: **15175962.8**

(22) Date of filing: **08.07.2015**

(54) **DETECTING CHROMOSOMAL ANEUPLOIDY**

NACHWEIS CHROMOSOMALER ANEUPLOIDIE

DÉTECTION DE L'ANEUPLOÏDIE CHROMOSOMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.07.2014 US 201462027258 P
03.03.2015 US 201514636193**

(43) Date of publication of application:
**27.01.2016 Bulletin 2016/04**

(73) Proprietor: **Yourgene Bioscience
221 New Taipei City (TW)**

(72) Inventor: **Chan, Chia-Han
238 New Taipei City (TW)**

(74) Representative: **Lang, Christian
LangPatent Anwaltskanzlei
IP Law Firm
Rosenheimer Straße 139
81671 München (DE)**

(56) References cited:
**WO-A1-2014/033455      WO-A2-2010/033578
AU-B2- 2013 203 079     US-A1- 2013 150 253**

• **A. J. SEHNERT ET AL: "Optimal Detection of Fetal
Chromosomal Abnormalities by Massively
Parallel DNA Sequencing of Cell-Free Fetal DNA
from Maternal Blood", CLINICAL CHEMISTRY,
vol. 57, no. 7, 1 July 2011 (2011-07-01), pages
1042-1049, XP55035090, ISSN: 0009-9147, DOI:
10.1373/clinchem.2011.165910**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Technical Field

The present disclosure relates to bioinformatics.

Description of Related Art

Aneuploidy is a condition in which the chromosome number is not an exact multiple of the number characteristic of a particular species. An extra or missing chromosome is a common cause of genetic disorders including human birth defects. Amniocentesis (also referred to as amniotic fluid test or AFT) is a medical procedure used in prenatal diagnosis of chromosomal abnormalities and fetal infections. The most common abnormalities detected are Down syndrome (trisomy 21), Edwards syndrome (trisomy 18), Patau syndrome (trisomy 13) and Turner syndrome (monosomy X). However, amniocentesis carries various risks, including miscarriage, needle injury, leaking amniotic fluid, Rh sensitization, infection, and infection transmission.

**SUMMARY**

The scope of the invention is defined by the appended claims. present invention, a method for detecting a chromosomal aneuploidy relating to a target nucleic acid region includes the following steps. A reference database is obtained. The reference database is established by sequencing a plurality of reference biological samples by a sequencing platform. At least one normalizing factor is determined based on the reference database. A cutoff value is determined based on the reference database. A biological sample under test is sequenced by the sequencing platform to obtain a number of target reads of the biological sample under test. The biological sample under test is obtained from a pregnant female and has nucleic acid molecules from the pregnant female and a fetus thereof. The target reads of the biological sample under test originate from the target nucleic acid region. The number of the target reads of the biological sample under test is normalized by the normalizing factor. The normalized number of the target reads of the biological sample under test is compared with the cutoff value. Whether the chromosomal aneuploidy relating to the target nucleic acid region is present in the fetus is determined based on the comparison.

In one example, the reference database can be gender based.

In one example, the determining the normalizing factor further includes the following steps. A number of target reads of each reference biological sample is determined, wherein the target reads of each reference biological sample originate from the target nucleic acid region. A number of correlated reads of each reference biological sample is determined, wherein the numbers of the correlated reads of the reference biological samples correlate with the numbers of the target reads of the reference biological samples, and the correlated reads of each reference biological sample originate from a correlated nucleic acid region. The normalizing factor is determined based on the numbers of the target reads of the reference biological samples and the numbers of the correlated reads of the reference biological samples.

In one example, the numbers of the correlated reads of the reference biological samples can linearly correlate with the numbers of the target reads of the reference biological samples.

In one example, the numbers of the correlated reads of the reference biological samples and the numbers of the target reads of the reference biological samples can have a correlation coefficient in a range from about 0.7 to about 0.99.

In one example, the determining the normalizing factor based on the numbers of the target reads of the reference biological samples and the numbers of the correlated reads of the reference biological samples further includes the following steps. Reference target local frequencies are determined, wherein each reference target local frequency is a ratio of the number of the target reads of each reference biological sample to a number of local reads of said each reference biological sample, and the local reads of each reference biological sample originate from the target nucleic acid region's own chromosome. Reference correlated global frequencies are determined, wherein each reference correlated global frequency is a ratio of the number of the correlated reads of each reference biological sample to a number of total reads of said each reference biological sample. A first correspondence between the reference target local frequencies and the reference correlated global frequencies is determined.

In one example, the normalizing the number of the target reads of the biological sample under test further includes the following steps. A test target global frequency is determined, wherein the test target global frequency is a ratio of the number of the target reads of the biological sample under test to a number of total reads of the biological sample under test. A test target local frequency is determined, wherein the test target local frequency is a ratio of the number of the target reads of the biological sample under test to a number of local reads of the biological sample under test, and the local reads of the biological sample under test originate from the target nucleic acid region's own chromosome. A test correlated global frequency is determined, wherein the test correlated global frequency is a ratio of a number of correlated reads of the biological sample under test to the number of the total reads of the biological sample under test, and the correlated reads of the biological sample under test originate from the correlated nucleic acid region. The test target local frequency is normalized by the test correlated global frequency and the first correspondence. The test target global frequency is normalized by the normalized test target local frequency.

In one example, the determining the normalizing factor based on the numbers of the target reads of the reference biological samples and the numbers of the correlated reads of the reference biological samples further includes the following steps. Reference target global frequencies are determined; wherein each reference target global frequency is a ratio of the number of the target reads of each reference biological sample to the number of the total reads of said each reference biological sample. The reference target local frequencies are respectively normalized by the reference correlated global frequencies and the first correspondence. The reference target global frequencies are respectively normalized by the normalized reference target local frequencies. A second correspondence between the normalized reference target global frequencies and the reference correlated global frequencies are determined.

In one example, the determining the cutoff value further includes the following steps. Reference estimated values are respectively estimated based on the reference correlated global frequencies and the second correspondence. Reference difference values between the normalized reference target global frequencies and the reference estimated values are respectively determined. The reference difference values to reference standard scores are respectively standardized based on the reference database. The cutoff value is determined based on the reference standard scores.

In one example, the normalizing the number of the target reads of the biological sample under test further includes the following steps. A test estimated value is estimated based on the test correlated global frequency and the second correspondence. A test difference value between the normalized test target global frequency and the test estimated value is determined. The test difference value to a test standard score is standardized based on the reference database, wherein the comparing includes comparing the test standard score with the cutoff value.

According to some embodiments of the present invention, a non-transitory machine readable medium stores a program which, when executed by at least one processing unit, detects a chromosomal aneuploidy relating to a target nucleic acid region. The program includes sets of instructions for the following steps. A reference database is obtained. The reference database is established by sequencing a plurality of reference biological samples by a sequencing platform. At least one normalizing factor is determined based on the reference database. A cutoff value is determined based on the reference database. A biological sample under test is sequenced by the sequencing platform to obtain a number of target reads of the biological sample under test. The biological sample under test is obtained from a pregnant female and has nucleic acid molecules from the pregnant female and a fetus thereof. The target reads of the biological sample under test originate from the target nucleic acid region. The number of the target reads of the biological sample under test is normalized by the normalizing factor. The normalized number of the target reads of the biological sample under test is compared with the cutoff value. Whether the chromosomal aneuploidy relating to the target nucleic acid region is present in the fetus is determined based on the comparison.

In one example, the reference database can be gender based.

In one example, the set of instructions for determining the normalizing factor includes sets of instructions for the following steps. A number of target reads of each reference biological sample is determined, wherein the target reads of each reference biological sample originate from the target nucleic acid region. A number of correlated reads of each reference biological sample is determined, wherein the numbers of the correlated reads of the reference biological samples correlate with the numbers of the target reads of the reference biological samples, and the correlated reads of each reference biological sample originate from a correlated nucleic acid region. The normalizing factor is determined based on the numbers of the target reads of the reference biological samples and the numbers of the correlated reads of the reference biological samples.

In one example, the numbers of the correlated reads of the reference biological samples can linearly correlate with the numbers of the target reads of the reference biological samples.

In one example, the numbers of the correlated reads of the reference biological samples and the numbers of the target reads of the reference biological samples can have a correlation coefficient in a range from about 0.7 to about 0.99.

In one example, the set of instructions for determining the normalizing factor based on the numbers of the target reads of the reference biological samples and the numbers of the correlated reads of the reference biological samples comprises sets of instructions for the following steps. Reference target local frequencies are determined, wherein each reference target local frequency is a ratio of the number of the target reads of each reference biological sample to a number of local reads of said each reference biological sample, and the local reads of each reference biological sample originate from the target nucleic acid region's own chromosome. Reference correlated global frequencies are determined, wherein each reference correlated global frequency is a ratio of the number of the correlated reads of each reference biological sample to a number of total reads of said each reference biological sample. A first correspondence between the reference target local frequencies and the reference correlated global frequencies are determined.

In one example, the set of instructions for normalizing the number of the target reads of the biological sample under test comprises sets of instructions for the following steps. A test target global frequency is determined, wherein the test target global frequency is a ratio of the number of the target reads of the biological sample under test to a number of total reads of the biological sample under test. A test target local frequency is determined, wherein the test target local frequency is a ratio of the number of the target reads of the biological sample under test to a number of local reads of the biological sample under test, and the local reads of the biological sample under test originate from the target nucleic acid region's own chromosome. A test correlated global frequency is determined, wherein the test correlated global frequency is a ratio of a number of correlated reads of the biological sample under test to the number of the total reads of the biological sample under test, and the correlated reads of the biological sample under test originate from the correlated nucleic acid region. The test target local frequency is normalized by the test correlated global frequency and the first correspondence. The test target global frequency is normalized by the normalized test target local frequency.

In one example, the set of instructions for determining the normalizing factor based on the numbers of the target reads of the reference biological samples and the numbers of the correlated reads of the reference biological samples comprises sets of instructions for the following steps. Reference target global frequencies are determined; wherein each reference target global frequency is a ratio of the number of the target reads of each reference biological sample to the number of the total reads of said each reference biological sample. The reference target local frequencies are respectively normalized by the reference correlated global frequencies and the first correspondence. The reference target global frequencies are respectively normalized by the normalized reference target local frequencies. A second correspondence between the normalized reference target global frequencies and the reference correlated global frequencies are determined.

In one example, the set of instructions for determining the cutoff value comprises sets of instructions for the following steps. Reference estimated values are respectively estimated based on the reference correlated global frequencies and the second correspondence. Reference difference values between the normalized reference target global frequencies and the reference estimated values are respectively determined. The reference difference values to reference standard scores are respectively standardized based on the reference database. The cutoff value is determined based on the reference standard scores.

In one example, the set of instructions for normalizing the number of the target reads of the biological sample under test comprises sets of instructions for the following steps. A test estimated value is estimated based on the test correlated global frequency and the second correspondence. A test difference value between the normalized test target global frequency and the test estimated value is determined. The test difference value to a test standard score is standardized based on the reference database, wherein the set of instructions for comparing includes a set of instructions for comparing the test standard score with the cutoff value.

## BRIEF DESCRIPTION OF THE DRAWINGS

Fig. 1 is a flowchart of a method for detecting a chromosomal aneuploidy according to some embodiments of the present invention.

Figs. 2A and 2B show plots of chromosome reads of a plurality of reference biological samples sequenced by the same sequencing platform according to a plurality of working examples of the present invention.

Fig. 3 is a flowchart of the step 120 of Fig. 1.

Fig. 4 is a flowchart of the step 123 of Fig. 3.

Fig. 5 shows global frequencies distribution of chromosome 21 of the reference biological samples according to one working example of the present invention.

Fig. 6 shows a correspondence between the chromosome 22 global frequencies of the reference biological samples and the chromosome 21 43-45Mb local frequencies of the reference biological samples.

Fig. 7 shows a correspondence between the chromosome 22 global frequencies of the reference biological samples and the chromosome 21 43-45Mb global frequencies of the reference biological samples.

Fig. 8 shows a correspondence between the chromosome 22 global frequencies of the reference biological samples and the normalized chromosome 21 43-45Mb global frequencies of the reference biological samples.

Fig. 9 is a flowchart of the step 130 of Fig. 1.

Fig. 10 shows reference standard scores distribution according to the working example shown in Figs. 5-8.

Fig. 11 is a flowchart of the step 150 of Fig. 1.

Fig. 12 shows test and reference standard scores distribution according to the working example shown in Figs. 5-8 and 10.

## DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically depicted in order to simplify the drawings.

The term "biological sample" as used herein refers to any biological sample that is taken from a subject (e.g., a human, such as a pregnant female) and contains one or more nucleic acid molecule(s) of interest.

The term "nucleic acid" refers to deoxyribonucleic acid (DNA), ribonucleic acid (RNA), or a polymer thereof in either single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.*, degenerate codon substitutions), alleles, orthologs, single nucleotide polymorphisms (SNPs), and complementary sequences as well as the sequence explicitly indicated. The term nucleic acid is used interchangeably with gene, cDNA, mRNA, small noncoding RNA, micro RNA (miRNA), Piwi-interacting RNA, and short hairpin RNA (shRNA) encoded by a gene or locus.

The term "based on" as used herein means "based at least in part on" and refers to one value or result being used in the determination of another value or result, such as occurs in the relationship of an input of a method and the output of that method.

The term "chromosomal aneuploidy" as used herein means a variation in the quantitative number of a chromosome from that of a diploid genome. The variation may be a gain or a loss. It may involve the whole of one chromosome or a region of a chromosome.

The term "cutoff value" as used herein means a numerical value whose value is used to arbitrate between two or more states (*e.g.* abnormal and normal) of classification for a biological sample. For example, if a parameter is greater than the cutoff value, a first classification of the quantitative data is made (*e.g.* abnormal); or if the parameter is less than the cutoff value, a different classification of the quantitative data is made (*e.g.* normal).

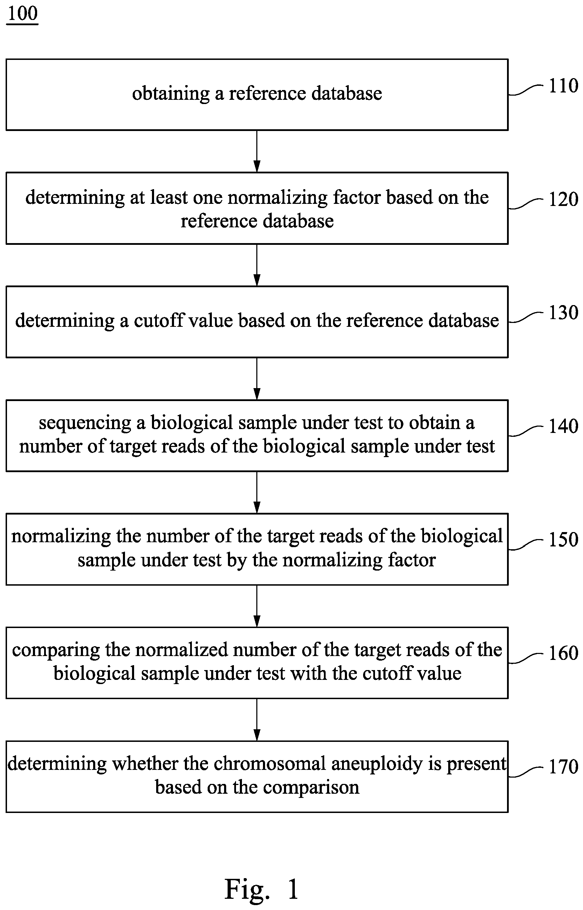

Fig. 1 is a flowchart of a method for detecting a chromosomal aneuploidy according to some embodiments of the present invention. As shown in Fig. 1, a method for detecting a chromosomal aneuploidy relating to a target nucleic acid region includes the following steps. In the step 110, a reference database is obtained. The reference database is established by sequencing a plurality of reference biological samples by a sequencing platform. The reference biological samples may be plasma, urine, serum, or any other suitable samples. The reference biological samples are respectively obtained from pregnant females. Therefore, each reference biological samples contains nucleic acid molecules from the pregnant female and a fetus thereof. The nucleic acid molecules may be, for example, fragments from chromosomes. The sequencing platform may be, for example, a next-generation sequencing platform, such as 454 platform (Roche), HiSeq, NextSeq, and MiSeq System (Illumina platform), Ion Torrent PGM and Proton System (Life Technologies), or Minion (Oxford Nanopore).

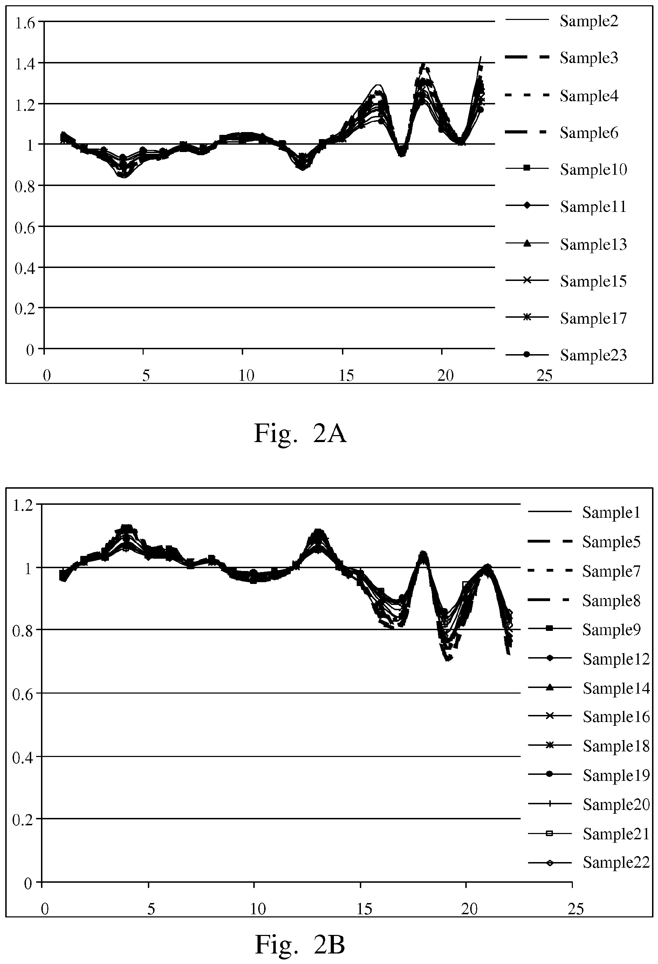

Figs. 2A and 2B shows plots of chromosome reads of a plurality of reference biological samples sequenced by the same sequencing platform according to a plurality of working examples of the present invention. In Figs. 2A and 2B, X axis is the chromosome number, and Y axis is the variation from the mean in a number of reads from each chromosome. As shown in Figs. 2A and 2B, there is a fluctuation in the number of the reads from each chromosome. This fluctuation may be caused by sample properties, system properties, or environmental factors. Therefore, whether the chromosomal aneuploidy is present in the fetus cannot be determined based on only the number of the reads from each chromosome because of the fluctuation.

Reference is made to Fig. 1. In the step 120, at least one normalizing factor is determined based on the reference database. As shown in Figs. 2A and 2B, the numbers of the reads from chromosomes correlate with each other. That is, an increase in the number of the reads from one chromosome would decrease the number of the reads from another chromosome. Since the reference biological samples of Figs. 2A and 2B were obtained in substantially the same way and were sequenced by the same sequencing platform, the fluctuations for different reference biological samples are substantially the same. Therefore, the correlated chromosome may be used to normalize the chromosome of interest to eliminate the fluctuation.

Since a female fetus does not have chromosome Y, a biological sample from a female fetus would have a different chromosome reads profile when compared with that of a male fetus sample. Therefore, the reference database of the step 110 may be gender based. That is, if a biological sample under test is from a male fetus, a male fetus reference database is used. If the biological sample under test is from a female fetus, a female fetus reference database is used.

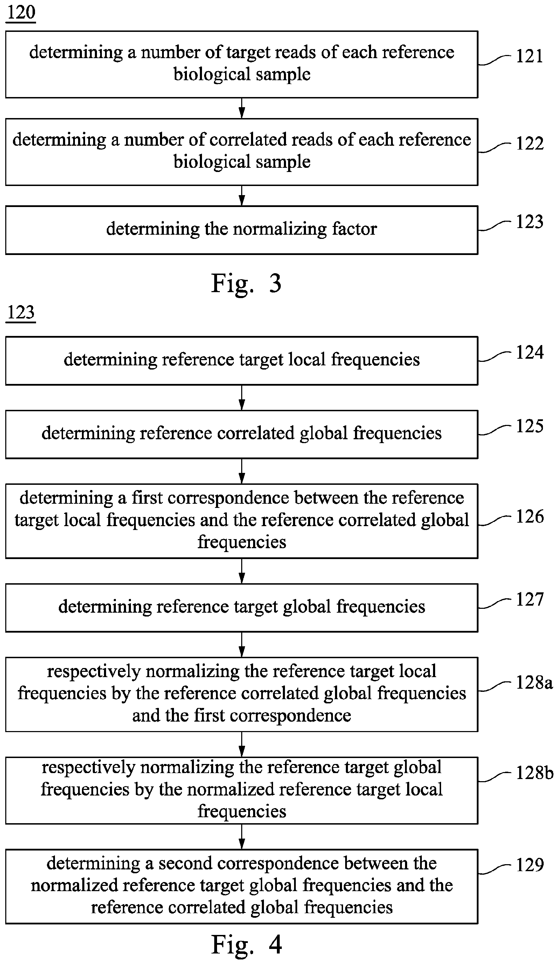

Fig. 3 is a flowchart of the step 120 of Fig. 1. The step 120 includes the following steps. In the step 121, a number of target reads of each reference biological sample is determined. The target reads of each reference biological sample originate from the target nucleic acid region. In the step 122, a number of correlated reads of each reference biological sample is determined. The numbers of the correlated reads of the reference biological samples correlate with the numbers of the target reads of the reference biological samples. For example, the numbers of the correlated reads of the reference biological samples may linearly correlate with the numbers of the target reads of the reference biological samples. The numbers of the correlated reads of the reference biological samples and the numbers of the target reads of the reference biological samples may have a correlation coefficient in a range from about 0.7 to about 0.99. The correlated reads of each reference biological sample originate from a correlated nucleic acid region. In the step 123, the normalizing factor is determined based on the numbers of the target reads of the reference biological samples and the numbers of the correlated reads of the reference biological samples.

Fig. 4 is a flowchart of the step 123 of Fig. 3. More specifically, the step 123 includes the following steps. In the step 124, reference target local frequencies are determined. Each reference target local frequency is a ratio of the number of the target reads of each reference biological sample to a number of local reads of said each reference biological sample. The local reads of each reference biological sample originate from the target nucleic acid region's own chromosome. In the step 125, reference correlated global frequencies are determined. Each reference correlated global frequency is a ratio of the number of the correlated reads of each reference biological sample to a number of total reads of said each reference biological sample. In the step 126, a first correspondence between the reference target local frequencies and the reference correlated global frequencies is determined. In the step 127, reference target global frequencies are determined. Each reference target global frequency is a ratio of the number of the target reads of each reference biological sample to the number of the total reads of said each reference biological sample. In the step 128a, the reference target local frequencies are respectively normalized by the reference correlated global frequencies and the first correspondence. In the step 128b, the reference target global frequencies are respectively normalized by the normalized reference target local frequencies. In the step 129, a second correspondence between the normalized reference target global frequencies and the reference correlated global frequencies is determined.

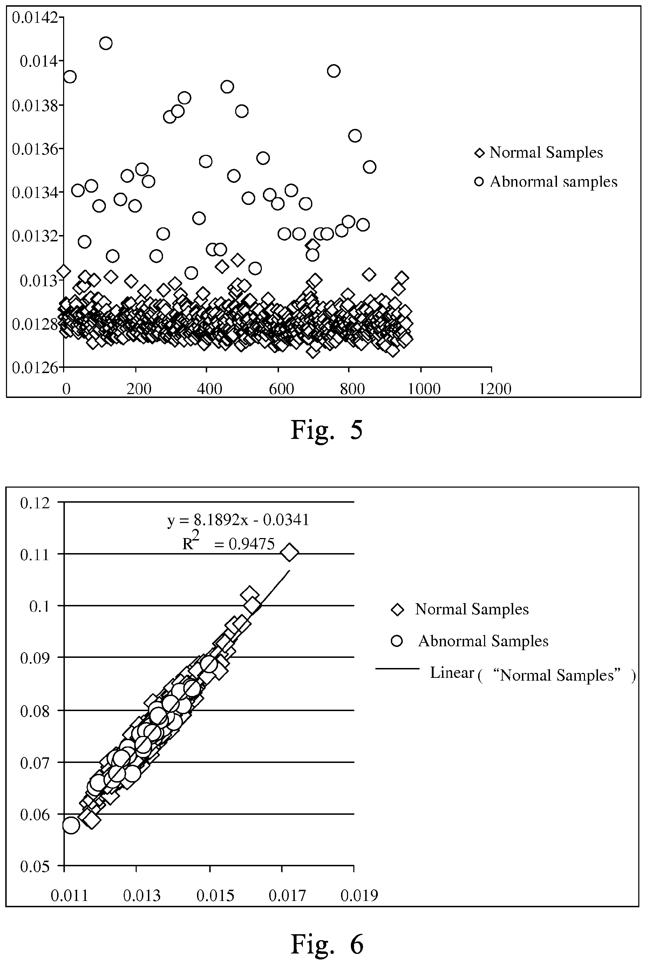

The following description will take male trisomy 21 detection as an example to illustrate how to perform the step 120. Fig. 5 shows global frequencies distribution of chromosome 21 for the reference biological samples according to one working example of the present invention. In Fig. 5, Y axis is the global frequency of chromosome 21. The global frequency of chromosome 21, r(21)/r(all), is a ratio of a number of reads of each reference biological sample originating from chromosome 21, r(21), to a number of total reads of said each reference biological sample, r(all). As shown in Fig. 5, some abnormal samples are mixed with normal samples, and therefore a cutoff value cannot be determined. In this working example of the present invention, the reference biological samples are 1004 in number, and the reference biological samples are plasma obtained from pregnant females. The sequencing platform is Illumina HiSeq.

In this working example of the present invention, chromosome 21 43-45Mb region was selected to be the target nucleic acid region. After inspection, it was found that numbers of reads of the reference biological samples originating from chromosome 22 and numbers of reads of the reference biological samples originating from chromosome 21 43-45Mb region have a correlation coefficient of 0.94. Therefore, chromosome 22 was selected to be the correlated nucleic acid region. According to the step 124, chromosome 21 43-45Mb local frequencies of the reference biological samples were determined. The chromosome 21 43-45Mb local frequency of each reference biological sample is a ratio of the number of the reads of said each reference biological sample originating from chromosome 21 43-45Mb region to a number of reads of said each reference biological sample originating from chromosome 21. According to the step 125, chromosome 22 global frequencies of the reference biological samples were determined. The chromosome 22 global frequency of each reference biological sample is a ratio of the number of the reads of said each reference biological sample originating from chromosome 22 to a number of total reads of said each reference biological sample.

Fig. 6 shows a correspondence between the chromosome 22 global frequencies of the reference biological samples and the chromosome 21 43-45Mb local frequencies of the reference biological samples. In Fig. 6, X axis is the chromosome 22 global frequency, and Y axis is the chromosome 21 43-45Mb local frequency. It was observed that there is a linear relationship between the chromosome 22 global frequencies of the reference biological samples which are normal and the chromosome 21 43-45Mb local frequencies of the reference biological samples which are normal. The chromosome 22 global frequencies of the reference biological samples which are normal and the chromosome 21 43-45Mb local frequencies of the reference biological samples which are normal have a coefficient of determination of 0.9475 and a regression line (Y=8.1892X-0.0341). The regression line (Y=8.1892X-0.0341) can be considered the first correspondence of the step 126.

According to the step 127, chromosome 21 43-45Mb global frequencies of the reference biological samples were determined. The chromosome 21 43-45Mb global frequency of each reference biological sample is a ratio of the number of the reads of said each reference biological sample originating from chromosome 21 43-45Mb region to the number of the total reads of said each reference biological sample.

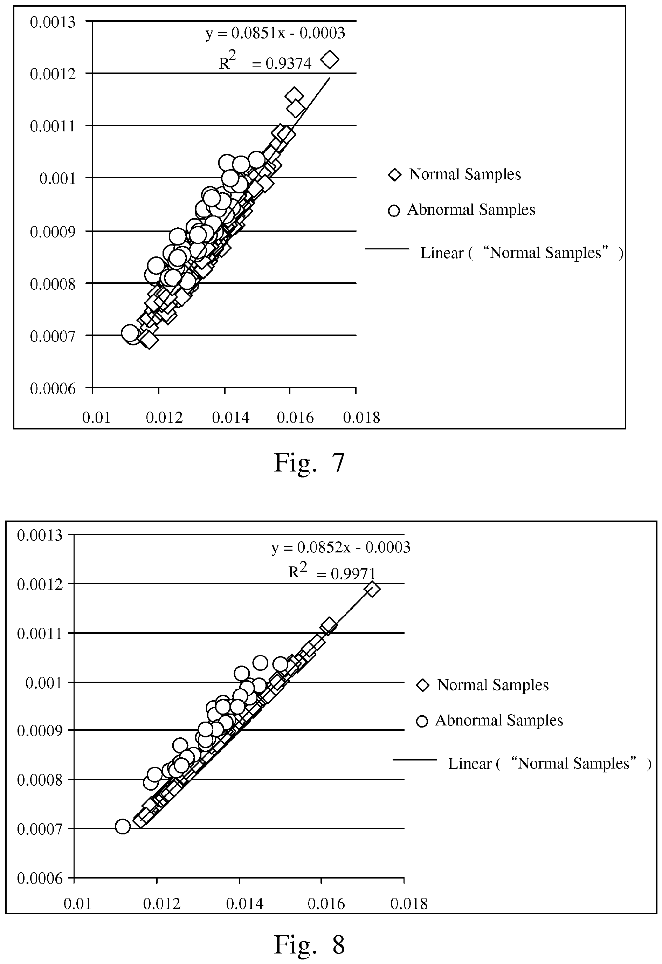

Fig. 7 shows a correspondence between the chromosome 22 global frequencies of the reference biological samples and the chromosome 21 43-45Mb global frequencies of the reference biological samples. In Fig. 7, X axis is the chromosome 22 global frequency, and Y axis is the chromosome 21 43-45Mb global frequency. It was observed that there is a linear relationship between the chromosome 22 global frequencies of the reference biological samples which are normal and the chromosome 21 43-45Mb global frequencies of the reference biological samples which are normal. The chromosome 22 global frequencies of the reference biological samples which are normal and the chromosome 21 43-45Mb global frequencies of the reference biological samples which are normal have a coefficient of determination of 0.9374 and a regression line (Y=0.0851X-0.0003).

According to the step 128a, the chromosome 21 43-45Mb local frequencies of the reference biological samples were respectively normalized by the chromosome 22 global frequencies of the reference biological samples and the first correspondence. According to the step 128b, the chromosome 21 43-45Mb global frequencies of the reference biological samples were respectively normalized by the normalized chromosome 21 43-45Mb local frequencies of the reference biological samples. Specifically, the normalized chromosome 21 43-45Mb global frequency of each reference biological sample was calculated by the following formula I:

$$y1=x3/(x2/(8.1892*x1-0.0341)) \dotfill \text{formula I}$$

, where y1 is the normalized chromosome 21 43-45Mb global frequency of each reference biological sample, x1 is the chromosome 22 global frequency of said each reference biological sample, x2 is the chromosome 21 43-45Mb local frequency of said each reference biological sample, and x3 is the chromosome 21 43-45Mb global frequency of said each reference biological sample.

Fig. 8 shows a correspondence between the chromosome 22 global frequencies of the reference biological samples and the normalized chromosome 21 43-45Mb global frequencies of the reference biological samples. In Fig. 8, X axis is the chromosome 22 global frequency, and Y axis is the normalized chromosome 21 43-45Mb global frequency. It was observed that there is a linear relationship between the chromosome 22 global frequencies of the reference biological samples which are normal and the normalized chromosome 21 43-45Mb global frequencies of the reference biological samples which are normal. The chromosome 22 global frequencies of the reference biological samples which are normal and the normalized chromosome 21 43-45Mb global frequencies of the reference biological samples which are normal have a coefficient of determination of 0.9971 and a regression line (Y=0.0852X-0.0003). The regression line (Y=0.0852X-0.0003) can be considered the second correspondence of the step 129.

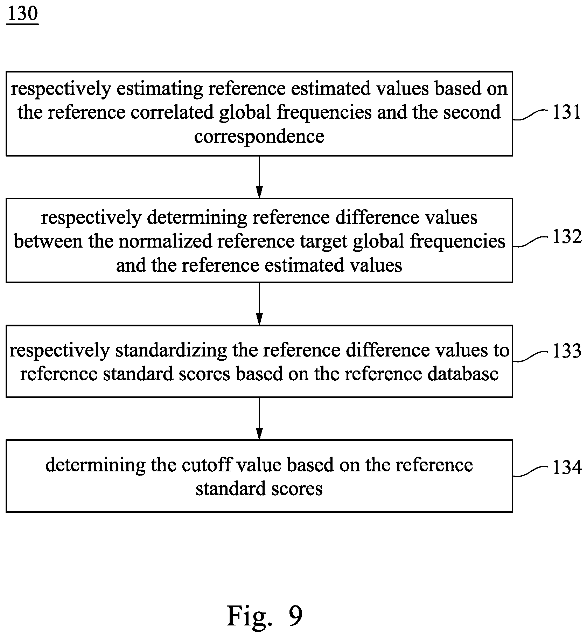

Reference is made to Fig. 1. In the step 130, a cutoff value is determined based on the reference database. Fig. 9 is a flowchart of the step 130 of Fig. 1. In the step 131, reference estimated values are respectively estimated based on the reference correlated global frequencies and the second correspondence. In the step 132, reference difference values between the normalized reference target global frequencies and the reference estimated values are respectively determined. In the step 133, the reference difference values are respectively standardized to reference standard scores based on the reference database. In the step 134, the cutoff value is determined based on the reference standard scores.

The following description will continue the male trisomy 21 detection shown in Figs. 5-8 to illustrate how to perform the step 130. In this working example, according to the step 131, reference estimated values were respectively estimated based on the chromosome 22 global frequencies of the reference biological samples and the second correspondence. According to the step 132, reference difference values were determined. Each reference difference value is between the normalized chromosome 21 43-45Mb global frequency of each reference biological sample and the corresponding reference estimated value. Specifically, each reference difference value was calculated by the following formula II:

$$y2=y1-(0.0852*x1-0.0003) \dotfill \text{formula II}$$

, where y1 is the normalized chromosome 21 43-45Mb global ratio of each reference biological sample, y2 is the reference difference value, and x1 is the chromosome 22 global frequency of said each reference biological sample.

According to the step 133, the reference difference values were respectively standardized to reference standard scores. Specifically, each reference standard score was calculated by the following formula III:

$$Z=y2-0.000028/0.0000094 \dots\dots\dots\dots\dots\dots\dots\dots\dots\dots\dots\dots\dots\dots\dots \text{formula III}$$

, where Z is the reference standard score, y2 is the reference difference value, 0.000028 is the average of the reference difference values, and 0.0000094 is the standard deviation of the reference difference values.

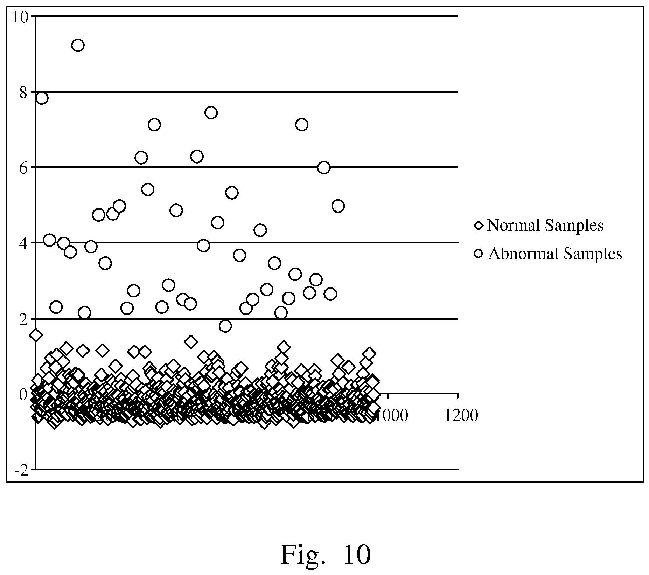

Fig. 10 shows reference standard scores distribution according to the working example shown in Figs. 5-8. In Fig. 10, Y axis is the standard score. As shown in Fig. 10, the abnormal samples are separated from the normal samples, and therefore a cutoff value could be determined accordingly. In the present working example, the cutoff value was determined to be 1.75.

Reference is made to Fig. 1. In the step 140, a biological sample under test is sequenced by the sequencing platform to obtain a number of target reads of the biological sample under test. The biological sample under test may be plasma, urine, serum, or any other suitable samples. The biological sample under test is obtained from a pregnant female and has nucleic acid molecules from the pregnant female and a fetus thereof. The nucleic acid molecules may be, for example, fragments from chromosomes. The target reads of the biological sample under test originate from the target nucleic acid region

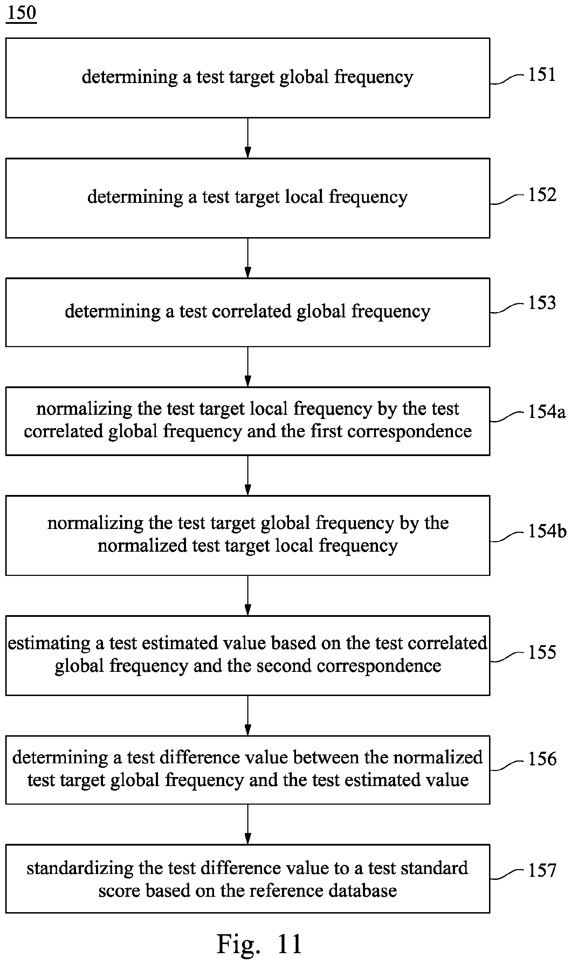

In the step 150, the number of the target reads of the biological sample under test is normalized by the normalizing factor. Fig. 11 is a flowchart of the step 150 of Fig. 1. In the step 151, a test target global frequency is determined. The test target global frequency is a ratio of the number of the target reads of the biological sample under test to a number of total reads of the biological sample under test. In the step 152, a test target local frequency is determined. The test target local frequency is a ratio of the number of the target reads of the biological sample under test to a number of local reads of the biological sample under test. The local reads of the biological sample under test originate from the target nucleic acid region's own chromosome. In the step 153, a test correlated global frequency is determined. The test correlated global frequency is a ratio of a number of correlated reads of the biological sample under test to the number of the total reads of the biological sample under test. The correlated reads of the biological sample under test originate from the correlated nucleic acid region. In the step 154a, the test target local frequency is normalized by the test correlated global frequency and the first correspondence. In the step 154b, the test target global frequency is normalized by the normalized test target local frequency.

In the step 155, a test estimated value is estimated based on the test correlated global frequency and the second correspondence. In the step 156, a test difference value between the normalized test target global frequency and the test estimated value is determined. In the step 157, the test difference value is standardized to a test standard score based on the reference database.

Reference is made to Fig. 1. In the step 160, the normalized number of the target reads of the biological sample under test is compared with the cutoff value. In the step 170, whether the chromosomal aneuploidy relating to the target nucleic acid region is present in the fetus is determined based on the comparison. Specifically, in the step 160, the test standard score calculated by the step 157 is compared with the cutoff value determined by the step 130. Then, in the step 170, if the test standard score calculated by the step 157 is greater than the cutoff value determined by the step 130, the chromosomal aneuploidy relating to the target nucleic acid region is determined to be present in the fetus, *i.e.* abnormal. If the test standard score calculated by the step 157 is less than the cutoff value determined by the step 130, the chromosomal aneuploidy relating to the target nucleic acid region is determined to be absent in the fetus, *i.e.* normal.

The following description will continue the male trisomy 21 detection shown in Figs. 5-8 and 10 to illustrate how to perform the steps 140-170. According to the step 140, a biological sample under test (plasma) was obtained from a pregnant female. Then, the biological sample under test was sequenced by Illumina HiSeq.

According to the step 151, a chromosome 21 43-45Mb global frequency of the biological sample under test was determined. The chromosome 21 43-45Mb global frequency of the biological sample under test is a ratio of a number of reads of the biological sample under test originating from chromosome 21 43-45Mb region to a number of total reads of the biological sample under test. According to the step 152, a chromosome 21 43-45Mb local frequency of the biological sample under test was determined. The chromosome 21 43-45Mb local frequency of the biological sample under test is a ratio of the number of the reads of the biological sample under test originating from chromosome 21 43-45Mb region to a number of reads of the biological sample under test originating from chromosome 21. According to the step 153, a chromosome 22 global frequency of the biological sample under test was determined. The chromosome 22 global frequency of the biological sample under test is a ratio of a number of reads of the biological sample under test originating from chromosome 22 to the number of the total reads of the biological sample under test.

According to the step 154a, the chromosome 21 43-45Mb local frequency of the biological sample under test was normalized by the chromosome 22 global frequency of the biological sample under test and the first correspondence.

In the step 154b, the chromosome 21 43-45Mb global frequency of the biological sample under test was normalized by the normalized chromosome 21 43-45Mb local frequency of the biological sample under test. Specifically, the normalized chromosome 21 43-45Mb global frequency of the biological sample under test was calculated by the following formula IV:

$$y1=x3/(x2/(8.1892*x1-0.0341))\ .......................................................\ \text{formula IV}$$

, where y1 is the normalized chromosome 21 43-45Mb global frequency of the biological sample under test, x1 is the chromosome 22 global frequency of the biological sample under test, x2 is the chromosome 21 43-45Mb local frequency of the biological sample under test, and x3 is the chromosome 21 43-45Mb global frequency of the biological sample under test.

According to the step 155, a test estimated value was estimated based on the chromosome 22 global frequency of the biological sample under test and the second correspondence. According to the step 156, a test difference value between the normalized chromosome 21 43-45Mb global frequency of the biological sample under test and the test estimated value was determined. Specifically, the test difference value was calculated by the following formula V:

$$y2=y1-(0.0852*x1-0.0003)\ ..............................................................\ \text{formula V}$$

, where y1 is the normalized chromosome 21 43-45Mb global frequency of the biological sample under test, y2 is the test difference value, x1 is the chromosome 22 global frequency of the biological sample under test.

According to the step 157, the test difference value was standardized to a test standard score based on the reference database. Specifically, the test standard score was calculated by the following formula VI:

$$Z=y2-0.000028/0.0000094\ .......................................................\ \text{formula VI}$$

, where Z is the test standard score, y2 is the test difference value, 0.000028 is the average of the reference difference values, and 0.0000094 is the standard deviation of the reference difference values.

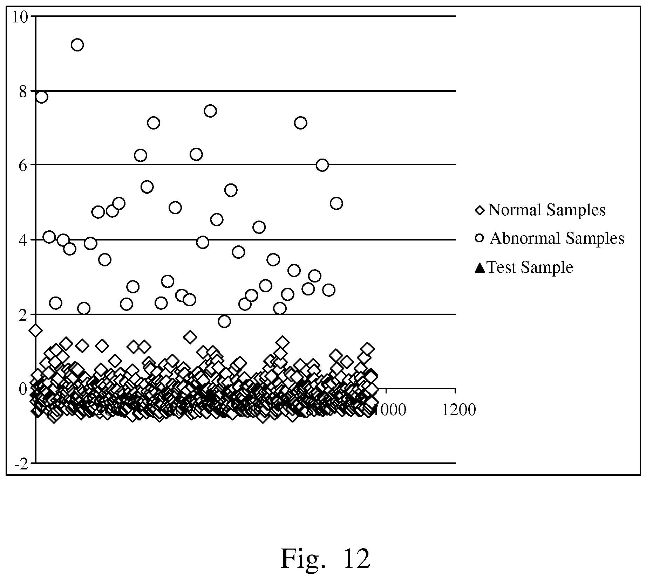

Fig. 12 shows test and reference standard scores distribution according to the working example shown in Figs. 5-8 and 10. In Fig. 12, Y axis is the standard score. As shown in Fig. 12, the test standard score is -0.16, which is less than the cutoff value, *i.e.* 1.75. Therefore, the biological sample under test was determined to be normal. That is, trisomy 21 was determined to be absent in the fetus.

In some embodiments, the method described above is implemented as a program stored in a non-transitory machine readable medium. When the program is executed by at least one processing unit, the method described above is performed. The non-transitory machine readable medium may include, but is not limited to, floppy disks, optical disks, compact discs (CDs), digital video discs (DVDs), magneto-optical disks, read-only memories (ROMs), random-access memories (RAMs), erasable programmable read only memories (EPROMs), electrically erasable programmable read-only memories (EEPROMs), magnetic or optical cards, or any type of media/machine readable medium suitable for storing instructions.

## Claims

1.   A method for detecting a chromosomal aneuploidy relating to a target nucleic acid region (100), the method comprising:

   obtaining a reference database (110), wherein the reference database is established by sequencing a plurality of reference biological samples by a sequencing platform;
   determining normalizing factors based on the reference database, wherein the determination of the normalizing factors (120) comprises:

   determining a number of target reads of each reference biological sample (121), wherein the target reads of each reference biological sample originate from the target nucleic acid region;
   determining a number of correlated reads of each reference biological sample (122), wherein the number of the correlated reads of the reference biological samples linearly correlate with the number of the target reads of the reference biological samples having a correlation coefficient in a range from about 0.7 to about

EP 2 977 466 B1

0.99, which is calculated by a linear regression model, and wherein the correlated reads of each reference biological sample originate from a correlated nucleic acid region; and

determining the normalizing factors based on the number of the target reads of the reference biological samples and the number of the correlated reads of the reference biological samples (123), wherein the normalizing factors comprise:

> a first correspondence between the reference target local frequencies and the reference correlated global frequencies and a test correlated global frequency for normalizing a test target local frequency, wherein the test correlated global frequency is a ratio of a number of correlated reads of the biological sample under test to the number of the total reads of the biological sample under test, and the correlated reads of the biological sample under test originate from the correlated nucleic acid region, and the test target local frequency is a ratio of the number of the target reads of the biological sample under test to a number of local reads of the biological sample under test, and the local reads of the biological sample under test originate from the target nucleic acid region's own chromosome; and
>
> a normalized test target local frequency for normalizing a test target global frequency, wherein the test target global frequency is a ratio of the number of the target reads of the biological sample under test to a number of total reads of the biological sample under test;

determining a cutoff value based on the reference database (130);

sequencing a biological sample under test by the sequencing platform to obtain a number of target reads of the biological sample under test (140), wherein the biological sample under test is obtained from a pregnant female and has nucleic acid molecules from the pregnant female and a fetus thereof, and the target reads of the biological sample under test originate from the target nucleic acid region;

normalizing the number of the target reads of the biological sample under test by the normalizing factors (150);

comparing the normalized number of the target reads of the biological sample under test with the cutoff value (160); and

determining whether the chromosomal aneuploidy relating to the target nucleic acid region is present in the fetus based on the comparison (170).

2. The method of claim 1, wherein the reference database is gender based.

3. The method of claim 1, wherein the determining the normalizing factors based on the numbers of the target reads of the reference biological samples and the numbers of the correlated reads of the reference biological samples (123) comprises:

> determining reference target local frequencies (124), wherein each reference target local frequency is a ratio of the number of the target reads of each reference biological sample to a number of local reads of said each reference biological sample, and the local reads of each reference biological sample originate from the target nucleic acid region's own chromosome;
>
> determining reference correlated global frequencies (125), wherein each reference correlated global frequency is a ratio of the number of the correlated reads of each reference biological sample to a number of total reads of said each reference biological sample; and
>
> determining the first correspondence between the reference target local frequencies and the reference correlated global frequencies (126).

4. The method of claim 3, wherein normalizing the number of the target reads of the biological sample under test (150) comprises:

> determining the test target global frequency (127);
>
> determining the test target local frequency (124);
>
> determining the test correlated global frequency (125);
>
> normalizing the test target local frequency by the test correlated global frequency and the first correspondence (128a); and
>
> normalizing the test target global frequency by the normalized test target local frequency (128b).

5. The method of claim 4, wherein the determining the normalizing factors based on the numbers of the target reads of the reference biological samples and the numbers of the correlated reads of the reference biological samples (123) comprises:

determining reference target global frequencies (127), wherein each reference target global frequency is a ratio of the number of the target reads of each reference biological sample to the number of the total reads of said each reference biological sample;

respectively normalizing the reference target local frequencies by the reference correlated global frequencies and the first correspondence (128a);

respectively normalizing the reference target global frequencies by the normalized reference target local frequencies (128b); and

determining a second correspondence between the normalized reference target global frequencies and the reference correlated global frequencies (129).

6. The method of claim 5, wherein the determining the cutoff value (130) comprises:

respectively estimating reference estimated values based on the reference correlated global frequencies and the second correspondence (131);

respectively determining reference difference values between the normalized reference target global frequencies and the reference estimated values (132);

respectively standardizing the reference difference values to reference standard scores based on the reference database (133); and

determining the cutoff value based on the reference standard scores (134).

7. The method of claim 6, wherein the normalizing the number of the target reads of the biological sample under test (150) comprises:

estimating a test estimated value based on the test correlated global frequency and the second correspondence (155);

determining a test difference value between the normalized test target global frequency and the test estimated value (156); and

standardizing the test difference value to a test standard score based on the reference database (157); wherein the comparing comprises:

comparing the test standard score with the cutoff value.

8. A non-transitory machine readable medium storing a program which, when executed by at least one processing unit, detects a chromosomal aneuploidy relating to a target nucleic acid region, the program comprising sets of instructions for:

obtaining a reference database (110), wherein the reference database is established by sequencing a plurality of reference biological samples by a sequencing platform;

determining normalizing factors based on the reference database (120) wherein the set of instructions for determining the normalizing factors (120) comprises sets of instructions for:

determining a number of target reads of each reference biological sample (121), wherein the target reads of each reference biological sample originate from the target nucleic acid region;

determining a number of correlated reads of each reference biological sample (122), wherein the number of the correlated reads of the reference biological samples linearly correlate with the number of the target reads of the reference biological samples having a correlation coefficient in a range from about 0.7 to about 0.99, which is calculated by a linear regression model, and wherein the correlated reads of each reference biological sample originate from a correlated nucleic acid region; and

determining the normalizing factors based on the number of the target reads of the reference biological samples and the number of the correlated reads of the reference biological samples (123), wherein the normalizing factors comprise:

a first correspondence between the reference target local frequencies and the reference correlated global frequencies and a test correlated global frequency for normalizing a test target local frequency, wherein the test correlated global frequency is a ratio of a number of correlated reads of the biological sample under test to the number of the total reads of the biological sample under test, and the correlated reads of the biological sample under test originate from the correlated nucleic acid region, and the test target local frequency is a ratio of the number of the target reads of the biological sample under test to a number of local reads of the biological sample under test, and the local reads of the biological sample under test originate from the target nucleic acid region's own chromosome; and

a normalized test target local frequency for normalizing a test target global frequency, wherein the test target global frequency is a ratio of the number of the target reads of the biological sample under test to a number of total reads of the biological sample under test;

determining a cutoff value based on the reference database (130);

sequencing a biological sample under test by the sequencing platform to obtain a number of target reads of the biological sample under test (140), wherein the biological sample under test is obtained from a pregnant female and has nucleic acid molecules from the pregnant female and a fetus thereof, and the target reads of the biological sample under test originate from the target nucleic acid region;

normalizing the number of the target reads of the biological sample under test by the normalizing factors (150);

comparing the normalized number of the target reads of the biological sample under test with the cutoff value (160); and

determining whether the chromosomal aneuploidy relating to the target nucleic acid region is present in the fetus based on the comparison (170).

9. The non-transitory machine readable medium of claim 8, wherein the reference database is gender based.

10. The non-transitory machine readable medium of claim 8, wherein the set of instructions for determining the normalizing factors based on the numbers of the target reads of the reference biological samples and the numbers of the correlated reads of the reference biological samples (123) comprises sets of instructions for:

determining reference target local frequencies (124), wherein each reference target local frequency is a ratio of the number of the target reads of each reference biological sample to a number of local reads of said each reference biological sample, and the local reads of each reference biological sample originate from the target nucleic acid region's own chromosome;

determining reference correlated global frequencies (125), wherein each reference correlated global frequency is a ratio of the number of the correlated reads of each reference biological sample to a number of total reads of said each reference biological sample; and

determining the first correspondence between the reference target local frequencies and the reference correlated global frequencies (126).

11. The non-transitory machine readable medium of claim 10, wherein the set of instructions for normalizing the number of the target reads of the biological sample under test (150) comprises sets of instructions for:

determining the test target global frequency (127);

determining the test target local frequency (124);

determining the test correlated global frequency (125);

normalizing the test target local frequency by the test correlated global frequency and the first correspondence (128a); and

normalizing the test target global frequency by the normalized test target local frequency (128b).

12. The non-transitory machine readable medium of claim 11, wherein the set of instructions for determining the normalizing factors based on the numbers of the target reads of the reference biological samples and the numbers of the correlated reads of the reference biological samples (123) comprises sets of instructions for:

determining reference target global frequencies (127), wherein each reference target global frequency is a ratio of the number of the target reads of each reference biological sample to the number of the total reads of said each reference biological sample;

respectively normalizing the reference target local frequencies by the reference correlated global frequencies and the first correspondence (128a);

respectively normalizing the reference target global frequencies by the normalized reference target local frequencies (128b); and

determining a second correspondence between the normalized reference target global frequencies and the reference correlated global frequencies (129).

13. The non-transitory machine readable medium of claim 12, wherein the set of instructions for determining the cutoff value (130) comprises sets of instructions for:

respectively estimating reference estimated values based on the reference correlated global frequencies and the second correspondence (131);

respectively determining reference difference values between the normalized reference target global frequencies and the reference estimated values (132);

respectively standardizing the reference difference values to reference standard scores based on the reference database (133); and

determining the cutoff value based on the reference standard scores (134).

14. The non-transitory machine readable medium of claim 13, wherein the set of instructions for normalizing the number of the target reads of the biological sample under test (150) comprises sets of instructions for:

estimating a test estimated value based on the test correlated global frequency and the second correspondence (155);

determining a test difference value between the normalized test target global frequency and the test estimated value (156); and

standardizing the test difference value to a test standard score based on the reference database (157);

wherein the set of instructions for comparing comprises a set of instructions for:

comparing the test standard score with the cutoff value.

## Patentansprüche

1. Verfahren zum Erkennen einer chromosomalen Aneuploidie bezüglich einer Zielnukleinsäureregion (100), wobei das Verfahren umfasst:

Erhalten einer Referenzdatenbank (110), wobei die Referenzdatenbank durch Sequenzierung einer Vielzahl von biologischen Referenzproben durch eine Sequenzierungsplattform erstellt wird;

Bestimmen von Normierungsfaktoren basierend auf der Referenzdatenbank, wobei die Bestimmung der Normierungsfaktoren (120) umfasst:

Bestimmen einer Anzahl von Ziellesevorgängen von jeder biologischen Referenzprobe (121), wobei die Ziellesevorgänge von jeder biologischen Referenzprobe der Zielnukleinsäureregion stammen;

Bestimmen einer Anzahl von korrelierten Lesevorgängen von jeder biologischen Referenzprobe (122), wobei die Anzahl der korrelierten Lesevorgänge der biologischen Referenzproben linear mit der Anzahl der Ziellesevorgänge der biologischen Referenzproben korreliert, die einen Korrelationskoeffizient in einem Bereich von etwa 0.7 bis etwa 0.99 aufweisen, was durch ein lineares Regressionsmodel berechnet wird, und wobei die korrelierten Lesevorgänge von jeder biologischen Referenzprobe von einer korrelierten Nukleinsäureregion stammen; und

Bestimmen der Normierungsfaktoren basierend auf der Anzahl von Ziellesevorgängen der biologischen Referenzproben und der Anzahl der korrelierten Lesevorgänge der biologischen Referenzproben (123), wobei die Normierungsfaktoren umfassen:

eine erste Übereinstimmung zwischen den Referenz-Ziellokalfrequenzen und den referenzkorrelierten Globalfrequenzen

und eine testkorrelierte Globalfrequenz zum

Normieren einer Test-Ziellokalfrequenz, wobei die testkorrelierte Globalfrequenz ein Verhältnis einer Anzahl korrelierter Lesevorgänge der getesteten biologischen Probe zu der Anzahl der gesamten Lesevorgänge der getesteten biologischen Probe ist und die korrelierten Lesevorgänge der getesteten biologischen Probe von der korrelierten Nukleinsäureregion stammen und die Test-Ziellokalfrequenz ein Verhältnis der Anzahl der Ziellesevorgänge der getesteten biologischen Probe zu einer Anzahl von lokalen Lesevorgängen der getesteten biologischen Probe ist und die lokalen Lesevorgänge der getesteten biologischen Probe aus dem eigenen Chromosom der Zielnukleinsäureregion stammen; und

eine normierte Test-Ziellokalfrequenz zum Normieren einer Test-Zielglobalfrequenz, wobei die Test-Zielglobalfrequenz ein Verhältnis der Anzahl der Ziellesevorgänge der getesteten biologischen Probe zu einer Anzahl von gesamten Lesevorgängen der getesteten biologischen Probe ist;

Bestimmen eines Cutoff-Werts basierend auf der Referenzdatenbank (130);

Sequenzieren einer getesteten biologischen Probe durch die Sequenzierungsplattform, um eine Anzahl von Ziellesevorgärigen der getesteten biologischen Probe (140) zu erhalten, wobei die getestete biologische Probe von einer schwangeren Frau bezogen wird und Nukleinsäuremoleküle der schwangeren Frau und eines Fötus derselben aufweist und die Ziellesevorgänge der getesteten biologischen Probe von der Zielnukleinsäureregion stammen;

Normieren der Anzahl der Ziellesevorgänge der getesteten biologischen Probe durch die Normierungsfaktoren (150);

Vergleichen der normierten Anzahl der Ziellesevorgänge der getesteten biologischen Probe mit dem Cutoff-Wert (160); und

basierend auf dem Vergleich (170) Bestimmen, ob die chromosomale Aneuploidie in Bezug auf die Zielnukleinsäureregion in dem Fötus vorhanden ist.

2. Verfahren nach Anspruch 1, wobei die Referenzdatenbank geschlechtsbasiert ist.

3. Verfahren nach Anspruch 1, wobei das Bestimmen der Normierungsfaktoren auf der Anzahl der Ziellesevorgänge der biologischen Referenzproben basiert und die Anzahl der korrelierten Lesevorgänge der biologischen Referenzproben (123) umfasst:

Bestimmen von Referenz-Ziellokalfrequenzen (124), wobei jede Referenz-Ziellokalfrequenz ein Verhältnis der Anzahl der Ziellesevorgänge jeder biologischen Referenzprobe zu einer Anzahl von lokalen Lesevorgängen jeder biologischen Referenzprobe ist und die lokalen Lesevorgänge jeder biologischen Referenzprobe aus dem eigenen Chromosom der Zielnukleinsäureregion stammen;

Bestimmen von referenzkorrelierten Globalfrequenzen (125), wobei jede referenzkorrelierte Globalfrequenz ein Verhältnis der Anzahl der korrelierten Lesevorgänge jeder biologischen Referenzprobe zu einer Anzahl von gesamten Lesevorgängen jeder biologischen Referenzprobe ist; und

Bestimmen der ersten Übereinstimmung zwischen den Referenz-Ziellokalfrequenzen und den referenzkorrelierten Globalfrequenzen (126).

4. Verfahren nach Anspruch 3, wobei das Normieren der Anzahl der Ziellesevorgänge der getesteten biologischen Probe (150) umfasst:

Bestimmen der Test-Zielglobalfrequenz (127);

Bestimmen der Test-Ziellokalfrequenz (124);

Bestimmen der testkorrelierten Globalfrequenz (125);

Normieren der Test-Ziellokalfrequenz durch die testkorrelierte Globalfrequenz und die erste Übereinstimmung (128a); und

Normieren der Test-Zielglobalfrequenz durch die normierte Test-Ziellokalfrequenz (128b).

5. Verfahren nach Anspruch 4, wobei das Bestimmen der Normierungsfaktoren basierend auf der Anzahl der Ziellesevorgänge der biologischen Referenzproben und der Anzahl der korrelierten Lesevorgänge der biologischen Referenzproben (123), umfasst:

Bestimmen von Referenz-Zielglobalfrequenzen (127), wobei jede Referenz-Zielglobalfrequenz ein Verhältnis der Anzahl der Ziellesevorgänge jeder biologischen Referenzprobe zu der Anzahl der gesamten Lesevorgänge jeder biologischen Referenzprobe ist;

jeweiliges Normieren der Referenz-Ziellokalfrequenzen durch die referenzkorrelierten Globalfrequenzen und die erste Übereinstimmung (128a);

jeweiliges Normieren der Referenz-Zielglobalfrequenzen durch die normierten Referenz-Ziellokalfrequenzen (128b); und

Bestimmen einer zweiten Übereinstimmung zwischen den normierten Referenz--Zielglobalfrequenzen und den referenzkorrelierten Globalfrequenzen (129).

6. Verfahren nach Anspruch 5, wobei das Bestimmen des Cutoff-Wertes (130) umfasst:

jeweiliges Abschätzen von Referenzschätzwerten basierend auf den referenzkorrelierten Globalfrequenzen und der zweiten Übereinstimmung (131);

jeweiliges Bestimmen von Referenzdifferenzwerten zwischen den normierten Referenz-Zielglobalfrequenzen und den Referenzschätzwerten (132);

jeweiliges Standardisieren der Referenzdifferenzwerte zu Referenzstandardpunktzahlen basierend auf der Referenzdatenbank (133); und

Bestimmen des Cutoff-Wertes basierend auf den Referenzstandardpunktzahlen (134).

7. Verfahren nach Anspruch 6, wobei das Normieren der Anzahl der Ziellesevorgänge der getesteten biologischen Probe (150) umfasst:

Schätzen eines Testschätzwertes basierend auf der testkorrelierten Globalfrequenz und der zweiten Übereinstimmung (155);
Bestimmen eines Testdifferenzwerts zwischen der normierten Test-Zielglobalfrequenz und dem Testschätzwert (156); und
Standardisieren des Testdifferenzwerts zu einer Teststandardpunktzahl basierend auf der Referenzdatenbank (157);
wobei das Vergleichen umfasst:
Vergleichen der Teststandardpunktzahl mit dem Cutoff-Wert.

8. Nichtvergängliches maschinenlesbares Medium, das ein Programm speichert, das, wenn es von mindestens einer Verarbeitungseinheit ausgeführt wird, eine chromosomale Aneuploidie in Bezug auf eine Zielnukleinsäureregion detektiert, wobei das Programm Sätze von Anweisungen umfasst für:

Erhalten einer Referenzdatenbank (110), wobei die Referenzdatenbank durch Sequenzieren mehrerer biologischer Referenzproben durch eine Sequenzierungsplattform erstellt wird;
Bestimmen von Normierungsfaktoren basierend auf der Referenzdatenbank (120), wobei der Satz von Anweisungen zum Bestimmen der Normierungsfaktoren (120) Sätze von Anweisungen umfasst für:

Bestimmen einer Anzahl von Ziellesevorgängen jeder biologischen Referenzprobe (121), wobei die Ziellesevorgänge jeder biologischen Referenzprobe aus der Zielnukleinsäureregion stammen;
Bestimmen einer Anzahl von korrelierten Lesevorgängen jeder biologischen Referenzprobe (122), wobei die Anzahl der korrelierten Lesevorgänge der biologischen Referenzproben linear mit der Anzahl der Ziellesevorgänge der biologischen Referenzproben mit einem Korrelationskoeffizienten in einem Bereich von ungefähr 0,7 bis etwa 0,99 korreliert, was durch ein lineares Regressionsmodell berechnet wird, und wobei die korrelierten Lesevorgänge jeder biologischen Referenzprobe aus einer korrelierten Nukleinsäureregion stammen; und
Bestimmen der Normierungsfaktoren basierend auf der Anzahl der Ziellesevorgänge der biologischen Referenzproben und der Anzahl der korrelierten Lesevorgänge der biologischen Referenzproben (123), wobei die Normierungsfaktoren umfassen:
eine erste Übereinstimmung zwischen den Referenz-Ziellokalfrequenzen und den referenzkorrelierten Globalfrequenzen

und eine testkorrelierte Globalfrequenz zum
Normieren einer Test-Ziellokalfrequenz, wobei die testkorrelierte Globalfrequenz ein Verhältnis einer Anzahl korrelierter Lesevorgänge der getesteten biologischen Probe zu der Anzahl der gesamten Lesevorgänge der getesteten biologischen Probe ist und die korrelierten Lesevorgänge der getesteten biologischen Probe aus der korrelierten Nukleinsäureregion stammen und die Test-Ziellokalfrequenz ein Verhältnis der Anzahl der Ziellesevorgänge der getesteten biologischen Probe zu einer Anzahl von lokalen Lesevorgängen der getesteten biologische Probe ist und die lokalen Lesevorgänge der getesteten biologischen Probe aus dem eigenen Chromosom der Zielnukleinsäureregion stammen; und
eine normierte Test-Ziellokalfrequenz zum Normieren einer Test-Zielglobalfrequenz, wobei die Test-Zielglobalfrequenz ein Verhältnis der Anzahl der Ziellesevorgänge der getesteten biologischen Probe zu einer Anzahl von gesamten Lesevorgängen der getesteten biologischen Probe aufweist;
Bestimmen eines Cutoff-Wertes basierend auf der Referenzdatenbank (130);
Sequenzieren einer getesteten biologischen Probe durch die Sequenzierungsplattform, um eine Anzahl von Ziellesevorgängen der getesteten biologischen Probe (140) zu erhalten, wobei die getestete biologische Probe von einer schwangeren Frau bezogen wird und Nukleinsäuremoleküle von der schwangeren Frau und eines Fötus derselben aufweist und die Zielwerte der getesteten biologischen Probe von der Zielnukleinsäureregion stammen;
Normieren der Anzahl der Ziellesevorgänge der getesteten biologischen Probe durch die Normierungsfaktoren (150);
Vergleichen der normierten Anzahl der Ziellesevorgänge der getesteten biologischen Probe mit dem Cutoff-Wert (160); und

basierend auf dem Vergleich (170) Bestimmen, ob die chromosomale Aneuploidie in Bezug auf die Zielnukleinsäureregion in dem Fötus vorhanden ist.

9. Nichtvergängliches maschinenlesbares Medium nach Anspruch 8, wobei die Referenzdatenbank geschlechterbasiert ist.

10. Nichtvergängliches maschinenlesbares Medium nach Anspruch 8, wobei der Satz von Anweisungen zum Bestimmen der Normierungsfaktoren basierend auf die Anzahl der Ziellesevorgänge der biologischen Referenzproben und der Anzahl der korrelierten Lesevorgänge der biologischen Referenzproben (123) Sätze von Anweisungen umfasst für:

Bestimmen von Referenz-Ziellokalfrequenzen (124), wobei jede Referenz-Ziellokalfrequenz ein Verhältnis der Anzahl der Ziellesevorgänge jeder biologischen Referenzprobe zu einer Anzahl von lokalen Lesevorgängen jeder biologischen Referenzprobe ist und die lokalen Lesevorgänge jeder biologischen Referenzprobe aus dem eigenen Chromosom der Zielnukleinsäureregion stammen;

Bestimmen von referenzkorrelierten Globalfrequenzen (125), wobei jede referenzkorrelierte Globalfrequenz ein Verhältnis der Anzahl der korrelierten Lesevorgänge jeder biologischen Referenzprobe zu einer Anzahl von gesamten Lesevorgängen jeder biologischen Referenzprobe ist; und

Bestimmen der ersten Übereinstimmung zwischen den Referenz-Ziellokalfrequenzen und den referenzkorrelierten Globalfrequenzen (126).

11. Nichtvergängliches maschinenlesbares Medium nach Anspruch 10, wobei der Satz von Anweisungen zum Normieren der Anzahl der Ziellesevorgänge der getesteten biologischen Probe (150) Sätze von Anweisungen umfasst für:

Bestimmen der Test-Zielglobalfrequenz (127);

Bestimmen der Test-Ziellokalfrequenz (124);

Bestimmen der testkorrelierten Globalfrequenz (125);

Normieren der Test-Ziellokalfrequenz durch die testkorrelierte Globalfrequenz und die erste Übereinstimmung (128a); und

Normieren der Test-Zielglobalfrequenz durch die normierte Test-Ziellokalfrequenz (128b).

12. Nichtvergängliches maschinenlesbares Medium nach Anspruch 11, wobei der Satz von Anweisungen zum Bestimmen der Normierungsfaktoren basierend auf der Anzahl der Ziellesevorgänge der biologischen Referenzproben und der Anzahl der korrelierten Lesevorgänge der biologischen Referenzproben (123) Sätze von Anweisungen umfasst für:

Bestimmen von Referenz-Zielglobalfrequenzen (127), wobei jede Referenz-Zielglobalfrequenz ein Verhältnis der Anzahl der Ziellesevorgänge jeder biologischen Referenzprobe zu der Anzahl der gesamten Lesevorgänge jeder biologischen Referenzprobe ist;

jeweiliges Normieren der Referenz-Ziellokalfrequenzen durch die referenzkorrelierten Globalfrequenzen und die erste Übereisttmmung (128a);

jeweiliges Normieren der Referenz-Zielglobalfrequenzen durch die normierten Referenz-Ziellokalfrequenzen (128b); und

Bestimmen einer zweiten Übereinstimmung zwischen den normierten Referenz-Zielglobalfrequenzen und den referenzkorrelierten Globalfrequenzen (129).

13. Nichtvergängliches maschinenlesbares Medium nach Anspruch 12, wobei der Satz von Anweisungen zum Bestimmen des Cutoff-Wertes (130) Sätze von Anweisungen umfasst für:

jeweiliges Abschätzen von Referenzschätzwerten basierend auf den referenzkorrelierten Globalfrequenzen und der zweiten Übereinstimmung (131);

jeweiliges Bestimmen von Referenzdifferenzwerten zwischen den normierten Referenz-Zielglobalfrequenzen und den Referenzschätzwerten (132);

Standardisierung der Referenzdifferenzwerte zu Referenzstandardpunktzahlen basierend auf der Referenzdatenbank (133); und

Bestimmen des Cutoff-Wertes basierend auf den Referenzstandardpunktzahlen (134).

14. Nichtvergängliches maschinenlesbares Medium nach Anspruch 13, wobei der Satz von Anweisungen zum Normieren der Anzahl der Ziellesevorgänge der getesteten biologischen Probe (150) Sätze von Anweisungen umfasst für:

Schätzen eines Testschätzwerts basierend auf der testkorrelierten Globalfrequenz und der zweiten Übereinstimmung (155);
Bestimmen eines Testdifferenzwerts zwischen der normierten Test-Zielglobalfrequenz und dem Testschätzwert (156); und
Standardisieren des Testdifferenzwerts zu einer Teststandardpunktzahl basierend auf der Referenzdatenbank (157);
wobei der Satz von Anweisungen zum Vergleichen einen Satz von Anweisungen umfasst für:
Vergleichen der Teststandardpunktzahl mit dem Cutoff-Wert.

## Revendications

1. Procédé pour détecter l'aneuploïdie chromosomique concernant une région d'acide nucléique cible (100), le procédé comprenant:

   l'obtention d'une base de données de référence (110), la base de données de référence étant établie en séquençant une pluralité d'échantillons biologiques de référence par une plateforme de séquençage ;
   la détermination de facteurs de normalisation basés sur la base de données de référence, la détermination des facteurs de normalisation (120) comprenant :

   la détermination d'un nombre de reads cibles de chaque échantillon biologique de référence (121), les reads cibles de chaque échantillon biologique de référence provenant de la région d'acide nucléique cible ;
   la détermination d'un nombre de reads corrélés de chaque échantillon biologique de référence (122), le nombre de reads corrélés des échantillons biologiques de référence étant corrélé linéairement avec le nombre de reads cibles des échantillons biologiques de référence ayant un coefficient de corrélation de l'ordre d'environ 0,7 à environ 0,99 qui est calculé par un modèle de régression linéaire et les reads corrélés de chaque échantillon biologique de référence provenant d'une région d'acide nucléique corrélé et
   la détermination des facteurs de normalisation basés sur le nombre de reads cibles des échantillons biologiques de référence et le nombre des reads corrélés des échantillons biologiques de référence (123), les facteurs de normalisation comprenant:

   une première correspondance entre les fréquences locales cibles et les fréquences globales corrélées de référence et une fréquence globale corrélée de test pour normaliser une fréquence locale cible de test, la fréquence globale corrélée de test étant un rapport d'un nombre de reads corrélés de l'échantillon biologique testé et du nombre de reads total de l'échantillon biologique testé et les reads corrélés de l'échantillon biologique testé provenant de la région d'acide nucléique corrélé et la fréquence locale cible test étant un rapport du nombre de reads cibles de l'échantillon biologique testé et des reads locaux de l'échantillon biologique testé provenant du chromosome propre à la région d'acide nucléique cible et
   une fréquence locale cible test normalisée pour normaliser une fréquence globale cible test, la fréquence globale cible test étant un rapport du nombre de reads cibles de l'échantillon biologique testé et d'un nombre de reads total de l'échantillon biologique testé;

   la détermination d'une valeur seuil basée sur la base de données de référence (130);
   le séquençage d'un échantillon biologique testé par la plateforme de séquençage pour obtenir un nombre de reads cibles de l'échantillon biologique testé (140), l'échantillon biologique testé étant obtenu d'une femme enceinte et ayant des molécules d'acide nucléique de la femme enceinte et du foetus de celle-ci et les reads cibles de l'échantillon biologique testé provenant de la région d'acide nucléique cible;
   la normalisation du nombre de reads cibles de l'échantillon biologique testé par les facteurs de normalisation (150);
   la comparaison du nombre normalisé des reads cibles de l'échantillon biologique testé avec la valeur seuil (160) et
   la détermination si l'aneuploïdie chromosomique concernant la région d'acide nucléique cible est présente dans le foetus sur la base de la comparaison (170).

2. Procédé selon la revendication 1, la base de données de référence étant basée sur le genre.

3. Procédé selon la revendication 1, la détermination des facteurs de normalisation basée sur les nombres des reads cibles des échantillons biologiques de référence et les nombres des reads corrélés des échantillons biologiques de référence (123) comprenant :

la détermination de fréquences locales cibles de référence (124), chaque fréquence locale cible de référence étant un rapport du nombre de reads cibles de chaque échantillon biologique de référence et d'un nombre de reads locaux de chaque échantillon biologique de référence mentionné et les reads locaux de chaque échantillon biologique de référence provenant du chromosome propre à la région d'acide nucléique cible ;

la détermination de fréquences globales corrélées de référence (125), chaque fréquence globale corrélée de référence étant un rapport du nombre de reads corrélés de chaque échantillon biologique de référence et d'un nombre de reads total dudit échantillon biologique de référence et

la détermination de la première correspondance entre les fréquences locales cibles de référence et les fréquences globales corrélées de référence (126).

4. Procédé selon la revendication 3, la normalisation du nombre des reads cibles de l'échantillon biologique testé (150) comprenant:

la détermination de la fréquence globale cible test (127);
la détermination de la fréquence locale cible test (124);
la détermination de la fréquence globale corrélée test (125);
la normalisation de la fréquence locale cible test par la fréquence globale corrélée test et la première correspondance (128a) et
la normalisation de la fréquence globale cible test par la fréquence locale cible test normalisée (128b).

5. Procédé selon la revendication 4, la détermination des facteurs de normalisation basée sur les nombres des reads cibles des échantillons biologiques de référence et les nombres des reads corrélés des échantillons biologiques de référence (123) comprenant :

la détermination des fréquences globales cibles de référence (127), chaque fréquence globale cible de référence étant un rapport du nombre de reads cibles de chaque échantillon biologique de référence et du nombre de reads total de chaque échantillon biologique de référence ;

respectivement la normalisation des fréquences locales cibles de référence par les fréquences globales corrélées de référence et la première correspondance (128a) ;

respectivement la normalisation des fréquences globales cibles de référence par les fréquences locales cibles de référence normalisées(128b) et

la détermination d'une seconde correspondance entre les fréquences globales cibles de référence normalisées et les fréquences globales corrélées de référence (129).

6. Procédé selon la revendication 5, la détermination de la valeur seuil (130) comprenant:

respectivement l'estimation des valeurs estimées de références basées sur les fréquences globales corrélées de référence et la seconde correspondance (131);

respectivement la détermination des valeurs de différence de référence entre les fréquences globales cibles de référence normalisées et les valeurs estimées de référence (132);

respectivement la standardisation des valeurs de différence de référence aux scores standard de référence basés sur la base de la base de données de référence (133) et

la détermination de la valeur seuil basée sur les scores standard de référence (134).

7. Procédé selon la revendication 6, la normalisation du nombre de reads cibles de l'échantillon biologique testé (150) comprenant:

l'estimation d'une valeur estimée test basée sur la fréquence globale corrélée test et la seconde correspondance (155);

la détermination d'une valeur de différence test entre la fréquence globale cible test normalisée et la valeur estimée test (156) et

la standardisation de la valeur de différence test à un score standard test basé sur la base de données de référence (157),

la comparaison comprenant:

la comparaison du score standard test avec la valeur seuil.

**8.** Support non éphémère lisible par machine stockant un programme qui, lorsqu'il est exécuté par au moins une unité de traitement, détecte une aneuploïdie chromosomique en relation avec une région d'acide nucléique cible, le programme comprenant des ensembles d'instructions pour:

l'obtention d'une base de données de référence (110), la base de données de référence étant établie en séquençant une pluralité d'échantillons biologiques de référence par une plateforme de séquençage;
la détermination de facteurs de normalisation basés sur la base de données de référence, l'ensemble d'instructions pour la détermination des facteurs de normalisation (120) comprenant:

la détermination d'un nombre de reads cibles de chaque échantillon biologique de référence (121), les reads cibles de chaque échantillon biologique de référence provenant de la région d'acide nucléique cible;
la détermination d'un nombre de reads corrélés de chaque échantillon biologique de référence (122), le nombre de reads corrélés des échantillons biologiques de référence étant corrélé linéairement avec le nombre de reads cibles des échantillons biologiques de référence ayant un coefficient de corrélation de l'ordre d'environ 0,7 à environ 0,99 qui est calculé par un modèle de régression linéaire et les reads corrélés de chaque échantillon biologique de référence provenant d'une région d'acide nucléique corrélée et
la détermination des facteurs de normalisation basés sur le nombre de reads cibles des échantillons biologiques de référence et le nombre des reads corrélés des échantillons biologiques de référence (123), les facteurs de normalisation comprenant :

une première correspondance entre les fréquences locales cibles et les fréquences globales corrélées de référence et une fréquence globale corrélée de test pour normaliser une fréquence locale cible de test, la fréquence globale corrélée de test étant un rapport d'un nombre de reads corrélés de l'échantillon biologique testé et du nombre de reads total de l'échantillon biologique testé et les reads corrélés de l'échantillon biologique testé provenant de la région d'acide nucléique corrélé et la fréquence locale cible test étant un rapport du nombre de reads cibles de l'échantillon biologique testé et des reads locaux de l'échantillon biologique testé provenant du chromosome propre à la région d'acide nucléique cible et
une fréquence locale cible test normalisée pour normaliser une fréquence globale cible test, la fréquence globale cible test étant un rapport du nombre de reads cibles de l'échantillon biologique testé et d'un nombre de reads total de l'échantillon biologique testé;

la détermination d'une valeur seuil basée sur la base de données de référence (130) ;
le séquençage d'un échantillon biologique testé par la plateforme de séquençage pour obtenir un nombre de reads cibles de l'échantillon biologique testé (140), l'échantillon biologique testé étant obtenu d'une femme enceinte et ayant des molécules d'acide nucléique de la femme enceinte et du foetus de celle-ci et les reads cibles de l'échantillon biologique testé provenant de la région d'acide nucléique cible ;
la normalisation du nombre de reads cibles de l'échantillon biologique testé par les facteurs de normalisation (150) ;
la comparaison du nombre normalisé des reads cibles de l'échantillon biologique testé avec la valeur seuil (160) et
la détermination si l'aneuploïdie chromosomique concernant la région d'acide nucléique cible est présente dans le foetus sur la base de la comparaison (170).

**9.** Support non éphémère lisible par machine selon la revendication 8, la base de données de référence étant basée sur le genre.

**10.** Support non éphémère lisible par machine selon la revendication 8, l'ensemble d'instructions pour déterminer les facteurs de normalisation basés sur les nombres de reads cibles des échantillons biologiques de référence et les nombres des reads corrélés des échantillons biologiques de référence (123) comprenant des ensembles d'instructions pour :

la détermination de fréquences locales cibles de référence (124), chaque fréquence locale cible de référence étant un rapport du nombre de reads cibles de chaque échantillon biologique de référence et d'un nombre de reads locaux de chaque échantillon biologique de référence mentionné et les reads locaux de chaque échantillon biologique de référence provenant du chromosome propre à la région d'acide nucléique cible ;
la détermination de fréquences globales corrélées de référence (125), chaque fréquence globale corrélée de référence étant un rapport du nombre de reads corrélés de chaque échantillon biologique de référence et d'un nombre de reads total dudit échantillon biologique de référence et
la détermination de la première correspondance entre les fréquences locales cibles de référence et les fréquences globales corrélées de référence (126).

**11.** Support non éphémère lisible par machine selon la revendication 10, l'ensemble d'instructions pour normaliser le nombre de reads cibles de l'échantillon biologique testé (150) comprenant des ensembles d'instructions pour :

la détermination de la fréquence globale cible test (127) ;
la détermination de la fréquence locale cible test (124) ;
la détermination de la fréquence globale corrélée test (125) ;
la normalisation de la fréquence locale cible test par la fréquence globale corrélée test et la première correspondance (128a) et
la normalisation de la fréquence globale cible test par la fréquence locale cible test normalisée (128b).

**12.** Support non éphémère lisible par machine selon la revendication 11, l'ensemble d'instructions pour déterminer les facteurs de normalisation sur la base des nombres des reads cibles des échantillons biologiques de référence et les nombres des reads corrélés des échantillons biologiques de référence (123) comprenant des ensembles d'instructions pour:

la détermination des fréquences globales cibles de référence (127), chaque fréquence globale cible de référence étant un rapport du nombre de reads cibles de chaque échantillon biologique de référence et du nombre de reads total de chaque échantillon biologique de référence;
respectivement la normalisation des fréquences locales cibles de référence par les fréquences globales corrélées de référence et la première correspondance (128a);
respectivement la normalisation des fréquences globales cibles de référence par les fréquences locales cibles de référence (128b) et
la détermination d'une seconde correspondance entre les fréquences globales cibles de référence normalisées et les fréquences globales corrélées de référence (129).

**13.** Support non éphémère lisible par machine selon la revendication 12, l'ensemble d'instructions pour déterminer la valeur seuil (130) comprenant des ensembles d'instructions pour:

respectivement l'estimation des valeurs estimées de références basées sur les fréquences globales corrélées de référence et la seconde correspondance (131);
respectivement la détermination des valeurs de différence de référence entre les fréquences globales cibles de référence normalisées et les valeurs estimées de référence (132);
respectivement la standardisation des valeurs de différence de référence aux scores standard de référence basés sur la base de la base de données de référence (133) et
la détermination de la valeur seuil basée sur les scores standard de référence (134).

**14.** Support non éphémère lisible par machine selon la revendication 13, l'ensemble d'instructions pour normaliser le nombre de reads cibles de l'échantillon biologique testé (150) comprenant des ensembles d'instructions pour :

l'estimation d'une valeur estimée test basée sur la fréquence globale corrélée test et la seconde correspondance (155);
la détermination d'une valeur de différence test entre la fréquence globale cible test normalisée et la valeur estimée test (156) et
la standardisation de la valeur de différence test à un score standard test basé sur la base de données de référence (157),
l'ensemble d'instructions pour la comparaison comprenant un ensemble d'instructions pour :
la comparaison du score standard test avec la valeur seuil.

<u>100</u>

```
┌─────────────────────────────────────────────────────┐
│          obtaining a reference database              │──── 110
└─────────────────────────────────────────────────────┘
                          │
                          ▼
┌─────────────────────────────────────────────────────┐
│    determining at least one normalizing factor       │
│          based on the reference database             │──── 120
└─────────────────────────────────────────────────────┘
                          │
                          ▼
┌─────────────────────────────────────────────────────┐
│  determining a cutoff value based on the reference   │──── 130
│                     database                         │
└─────────────────────────────────────────────────────┘
                          │
                          ▼
┌─────────────────────────────────────────────────────┐
│   sequencing a biological sample under test to       │
│   obtain a number of target reads of the             │──── 140
│        biological sample under test                  │
└─────────────────────────────────────────────────────┘
                          │
                          ▼
┌─────────────────────────────────────────────────────┐
│  normalizing the number of the target reads of the   │
│   biological sample under test by the normalizing    │──── 150
│                     factor                           │
└─────────────────────────────────────────────────────┘
                          │
                          ▼
┌─────────────────────────────────────────────────────┐
│ comparing the normalized number of the target reads  │
│  of the biological sample under test with the cutoff │──── 160
│                      value                           │
└─────────────────────────────────────────────────────┘
                          │
                          ▼
┌─────────────────────────────────────────────────────┐
│ determining whether the chromosomal aneuploidy is    │
│     present based on the comparison                  │──── 170
└─────────────────────────────────────────────────────┘
```

Fig. 1

Fig. 2A

Fig. 2B

120

determining a number of target reads of each reference biological sample — 121

determining a number of correlated reads of each reference biological sample — 122

determining the normalizing factor — 123

## Fig. 3

123

determining reference target local frequencies — 124

determining reference correlated global frequencies — 125

determining a first correspondence between the reference target local frequencies and the reference correlated global frequencies — 126

determining reference target global frequencies — 127

respectively normalizing the reference target local frequencies by the reference correlated global frequencies and the first correspondence — 128a

respectively normalizing the reference target global frequencies by the normalized reference target local frequencies — 128b

determining a second correspondence between the normalized reference target global frequencies and the reference correlated global frequencies — 129

## Fig. 4

Fig. 5

Fig. 6

Fig. 7

Fig. 8

130

respectively estimating reference estimated values based on the reference correlated global frequencies and the second correspondence ⟋ 131

respectively determining reference difference values between the normalized reference target global frequencies and the reference estimated values ⟋ 132

respectively standardizing the reference difference values to reference standard scores based on the reference database ⟋ 133

determining the cutoff value based on the reference standard scores ⟋ 134

Fig. 9

Fig. 10

150

```
┌─────────────────────────────────────────────────┐
│   determining a test target global frequency     │──── 151
└─────────────────────────────────────────────────┘
                        │
                        ▼
┌─────────────────────────────────────────────────┐
│   determining a test target local frequency      │──── 152
└─────────────────────────────────────────────────┘
                        │
                        ▼
┌─────────────────────────────────────────────────┐
│  determining a test correlated global frequency  │──── 153
└─────────────────────────────────────────────────┘
                        │
                        ▼
┌─────────────────────────────────────────────────┐
│ normalizing the test target local frequency by   │
│ the test correlated global frequency and the     │──── 154a
│ first correspondence                             │
└─────────────────────────────────────────────────┘
                        │
                        ▼
┌─────────────────────────────────────────────────┐
│ normalizing the test target global frequency by  │
│ the normalized test target local frequency       │──── 154b
└─────────────────────────────────────────────────┘
                        │
                        ▼
┌─────────────────────────────────────────────────┐
│ estimating a test estimated value based on the   │
│ test correlated global frequency and the second  │──── 155
│ correspondence                                   │
└─────────────────────────────────────────────────┘
                        │
                        ▼
┌─────────────────────────────────────────────────┐
│ determining a test difference value between the  │
│ normalized test target global frequency and the  │──── 156
│ test estimated value                             │
└─────────────────────────────────────────────────┘
                        │
                        ▼
┌─────────────────────────────────────────────────┐
│ standardizing the test difference value to a     │
│ test standard score based on the reference       │──── 157
│ database                                         │
└─────────────────────────────────────────────────┘
```

Fig. 11

Fig. 12